# EUROPEAN PATENT APPLICATION

(11) **EP 2 550 913 A2**
(43) Date of publication of application: **30.01.2013**
(21) Application number: 12005340.0
(22) Date of filing: 20.07.2012
(51) Int. Cl.: A61B 3/103, A61B 3/13, G02B 21/00, A61F 2/16

(54) **A microscope for ophthalmologic surgery**

(30) Priority: 29.07.2011 JP 2011166906
(71) Applicant: Kabushiki Kaisha Topcon, Tokyo 174-8580 (JP)
(72) Inventor: Nomura, Kazuhisa, Tokyo 174-8580 (JP); Kitajima, Nobuaki, Saitama, 349-0212 (JP); Sato, Toshiaki, Tokyo 174-8580 (JP)
(74) Representative: Gagel, Roland

(57) **Abstract**

A microscope for ophthalmologic surgery according to an embodiment comprises: an optical system that photographs a patient's eye; a main body part that stores at least part of the optical system; an attachment that is attached to the main body part and has multiple bright points arranged in a ring; a determination part that determines whether the eyelids of the patient's eye are open or closed by determining whether or not bright point images are depicted within a prescribed region in the frame of a first image obtained by using the optical system to photograph the patient's eye while the multiple bright points are projected thereto; and a calculation part that calculates astigmatism information of the patient's eye based on a second image obtained by using the optical system to photograph the patient's eye while the multiple bright points are projected thereto.

## Description

### [Field of the Invention]

The present invention relates to a microscope for ophthalmologic surgery.

### [Background of the Invention]

A microscope for ophthalmologic surgery, which can measure the astigmatic axis angles of a patient's eye and project the measurement results onto the patient's eye, is known (Refer to Japanese published unexamined application 2011-110172). The microscope for ophthalmologic surgery projects multiple bright points arranged in a circle onto the patient's eye, and calculates the astigmatic axis angle based on the arrangement of these projection images. Furthermore, by projecting bright points at positions corresponding to the calculated astigmatic axis direction to the patient's eye in a characteristic mode, this microscope for ophthalmologic surgery makes the astigmatic axis direction visible to the examiner.

Such a microscope for ophthalmologic surgery is suitable for transplant surgeries of toric IOL (Intraocular Lens) for correcting astigmatism. That is, to effectively correct astigmatism, not only the astigmatic degree but also the astigmatic axis angle (astigmatic axis direction) is important. Consequently, it is necessary to adjust the orientation of the lens with high accuracy when transplanting the toric IOL. The microscope for ophthalmologic surgery in Japanese published unexamined application 2011-110172 satisfies such needs. Furthermore, it is also advantageous in that the traditional method that directly marks the astigmatic axis angle on the patient's eye does not have to be followed.

### [Prior Art Documents]

### [Patent Documents]

[Patent Document 1] Japanese published unexamined application 2011-110172

### [Summary of the Invention]

### [Problem to be Solved by the Invention]

The microscope for ophthalmologic surgery of Japanese published unexamined application 2011-110172 measures the state of astigmatism by projecting bright points to the patient's eye. Consequently, the state of the cornea of the patient's eye, and particularly the state of the fluid (tear fluid, etc.) on the corneal surface, affects the corneal reflection of the bright points, creating the possibility that measurements may be performed incorrectly.

The present invention has been devised based on considerations of such facts, and the objective thereof is to provide a microscope for ophthalmologic surgery that makes it possible to perform astigmatism measurements correctly.

### [Means for Solving the Problem]

A microscope for ophthalmologic surgery according to Claim 1 comprises: an optical system that photographs a patient's eye; a main body part that stores at least part of the optical system; an attachment that is attached to the main body part and has multiple bright points arranged in a ring; a determination part that determines whether the eyelids of the patient's eye are open or closed by determining whether or not bright point images are depicted within a prescribed region in the frame of a first image obtained by using the optical system to photograph the patient's eye while the multiple bright points are projected thereto; and a calculation part that calculates astigmatism information of the patient's eye based on a second image obtained by using the optical system to photograph the patient's eye while the multiple bright points are projected thereto.
A microscope for ophthalmologic surgery according to Claim 2 comprises: an optical system that photographs a patient's eye; a main body part that stores at least part of the optical system; an attachment that is attached to the main body part and has multiple bright points arranged in a ring; a determination part that determines whether the eyelids of the patient's eye are open or closed by determining whether or not bright point images are depicted within a prescribed region in the frame of a first image obtained by using the optical system to photograph the patient's eye while the multiple bright points are projected thereto; and a calculation part that, when the determination part determines that the eyelids have shifted from a closed state to an open state and that the open-eyelid state has continued for a prescribed time, calculates astigmatism information of the patient's eye based on a second image obtained by using the optical system to photograph the patient's eye while the multiple bright points are projected thereto.
An invention according to Claim 3 is the microscope for ophthalmologic surgery according to Claim 1 or 2, wherein the prescribed regions include multiple linear regions arranged in parallel at a prescribed interval.
An invention according to Claim 4 is the microscope for ophthalmologic surgery according to Claim 3, wherein the prescribed interval is determined based on the positions at which the bright point images are depicted in the frame.
An invention according to Claim 5 is the microscope for ophthalmologic surgery according to Claim 3, wherein the optical system includes a variable magnification optical system that changes the imaging magnification, and the prescribed interval is determined based on the imaging magnification.
An invention according to Claim 6 is the microscope for ophthalmologic surgery according to any of Claim 1 through 5, wherein the determination part determines whether or not the bright point images are depicted based on the distribution of luminance values in the prescribed regions.
An invention according to Claim 7 is the microscope for ophthalmologic surgery according to Claim 6, wherein the determination part differentiates the distribution of the luminance values and determines whether or not the bright point images are depicted based on the distribution of the derivatives.
An invention according to Claim 8 is the microscope for ophthalmologic surgery according to any of Claims 1 through 7, wherein each of the multiple bright points outputs light including a wavelength of the visible region.
A microscope for ophthalmologic surgery according to Claim 9 comprises: an optical system that photographs a patient's eye; a main body part that stores at least part of the optical system; an attachment that is attached to the main body part and has multiple bright points arranged in a ring; a determination part that determines whether the eyelids of the patient's eye are open or closed based on images of the patient's eye photographed by the optical system; and a calculation part that, when the determination part determines that the eyelids have shifted from a closed state to an open state and that the open-eyelid state has continued for a prescribed time, calculates astigmatism information of the patient's eye based on an image obtained by using the optical system to photograph the patient's eye while the multiple bright points are projected thereto.
An invention according to Claim 10 is the microscope for ophthalmologic surgery according to Claim 2 or 9, further comprising a changing part for changing the prescribed time.
An invention according to Claim 11 is the microscope for ophthalmologic surgery according to Claim 2 or 9, further comprising: an operation part; and a mode switching part that switches the operation mode of the calculation part between a first mode that calculates the astigmatism information based on determination results from the determination part, and a second mode that calculates the astigmatism information in accordance with operations of the operation part.
An invention according to Claim 12 is the microscope for ophthalmologic surgery according to any of Claims 1 through 11, wherein the determination part performs the determination at a prescribed time interval, and the microscope for ophthalmologic surgery further comprises a notification part that performs notification when determination results indicating the closed-eyelid state are continuously obtained a prescribed number of times by the determination part.

### [Effect of the Invention]

According to the microscope for ophthalmologic surgery of the present invention, it is possible to determine whether or not the patient's eyelids are open and to perform astigmatism measurement of the patient's eye. Therefore, it is possible to perform astigmatism measurements correctly.

Moreover, according to the microscope for ophthalmologic surgery of the present invention, it is possible to perform astigmatism measurement automatically when it is determined that the patient's eyelids have shifted from a closed state to an open state and that the open-eyelid state has continued for a prescribed time. As a result, it is possible to perform astigmatism measurement automatically when the state of the cornea of the patient's eye, particularly the state of tear fluid, etc. on the corneal surface, does not substantially affect the corneal reflection of the bright points. Consequently, it is possible to perform astigmatism measurements correctly.

### [Brief Description of the Drawings]

Fig. 1 is a schematic drawing showing an example of the exterior configuration of the microscope for ophthalmologic surgery pertaining to the embodiment.
Fig. 2 is a schematic drawing showing an example of the configuration of a projection image forming part in the microscope for ophthalmologic surgery pertaining to the embodiment.
Fig. 3 is a schematic drawing showing an example of the configuration of a projection image forming part in the microscope for ophthalmologic surgery pertaining to the embodiment.
Fig. 4 is a schematic drawing showing an example of the configuration of an optical system in the microscope for ophthalmologic surgery pertaining to the embodiment.
Fig. 5 is a schematic drawing showing an example of the configuration of an optical system in the microscope for ophthalmologic surgery pertaining to the embodiment.
Fig. 6 is a schematic block diagram showing an example of the configuration of a control system in the microscope for ophthalmologic surgery pertaining to the embodiment.
Fig. 7 is an explanatory drawing of a processing executed by the microscope for ophthalmologic surgery pertaining to the embodiment.
Fig. 8 is an explanatory drawing of a processing executed by the microscope for ophthalmologic surgery pertaining to the embodiment.
Fig. 9 is an explanatory drawing of a processing executed by the microscope for ophthalmologic surgery pertaining to the embodiment.
Fig. 10 is an explanatory drawing of a processing executed by the microscope for ophthalmologic surgery pertaining to the embodiment.
Fig. 11 is an explanatory drawing of a processing executed by the microscope for ophthalmologic surgery pertaining to the embodiment.
Fig. 12 is an explanatory drawing of a processing executed by the microscope for ophthalmologic surgery pertaining to the embodiment.
Fig. 13 is a flowchart showing an example of an action of the microscope for ophthalmologic surgery pertaining to the embodiment.

### [Mode for Carrying Out the Invention]

An example of an embodiment of the microscope for ophthalmologic surgery, as related to the present invention, will be explained in detail with reference to the diagrams.

### [Configuration]

### [Exterior configuration]

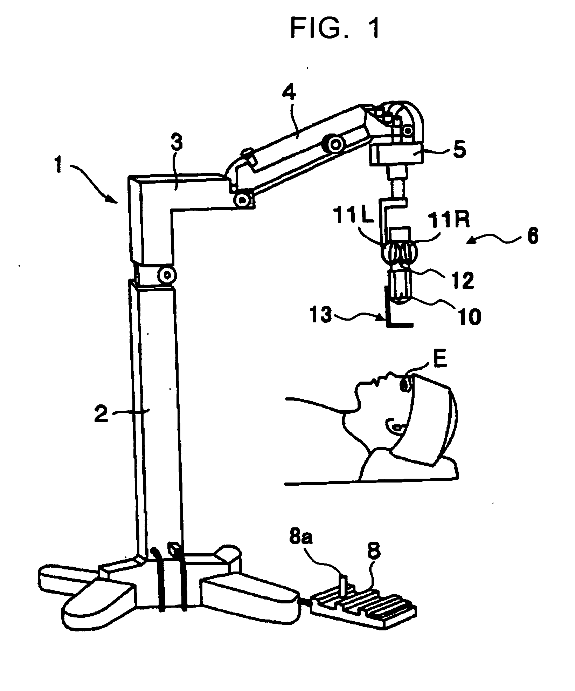

The exterior configuration of a microscope for ophthalmologic surgery 1, as related to the embodiment, will be explained with reference to Fig. 1. The microscope for ophthalmologic surgery 1 is composed of a pole 2, a first arm 3, a second arm 4, a driving device 5, a microscope 6, and a foot switch 8.

The driving device 5 is composed of an actuator such as a motor. The driving device 5 moves the microscope 6 up and down as well as horizontally, depending on the operation using an operating lever 8a of the foot switch 8. This makes it possible to move the microscope 6 3-dimensionally.

Various optical systems, drive systems, etc. are stored in a lens tube part 10 of the microscope 6. An inverter part 12 is provided in the top part of the lens tube part 10. When an observation image of a patient's eye is obtained as a reversed image, the inverter part 12 converts the observation image to an erect image. A left-and-right pair of eyepiece parts 11 Land 11R is provided at the top part of the inverter part 12. An observer (such as an operator) can view the patient's eye with the both eyes by looking into the eyepiece parts 11L and 11R. The microscope 6 is an example of the "main body."

The microscope for ophthalmologic surgery 1 has a projection image forming part 13. The projection image forming part 13 is removable from the microscope 6 and forms a specified projection image on the patient's eye E by projecting luminous flux onto the patient's eye E. The projection image forming part 13 is an example of an "attachment."

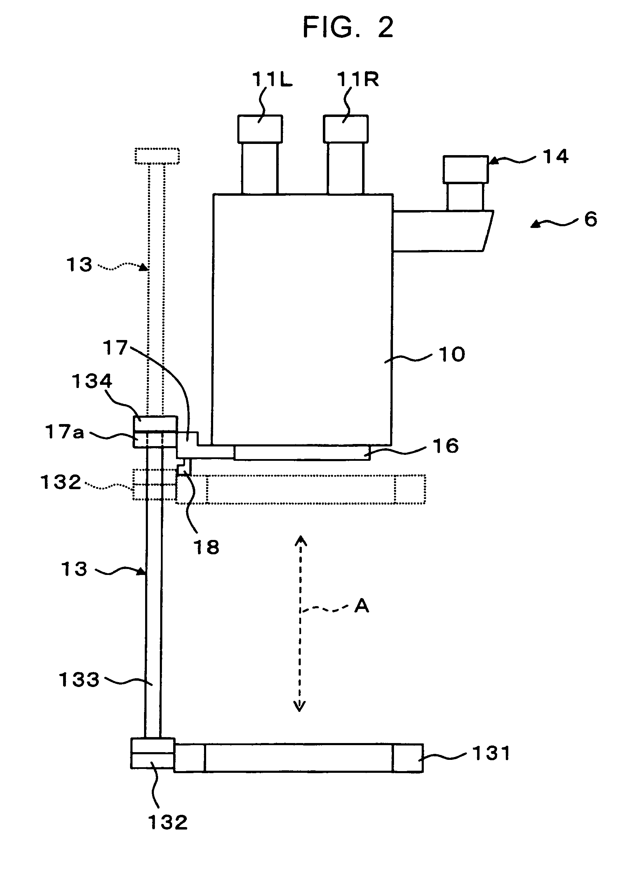

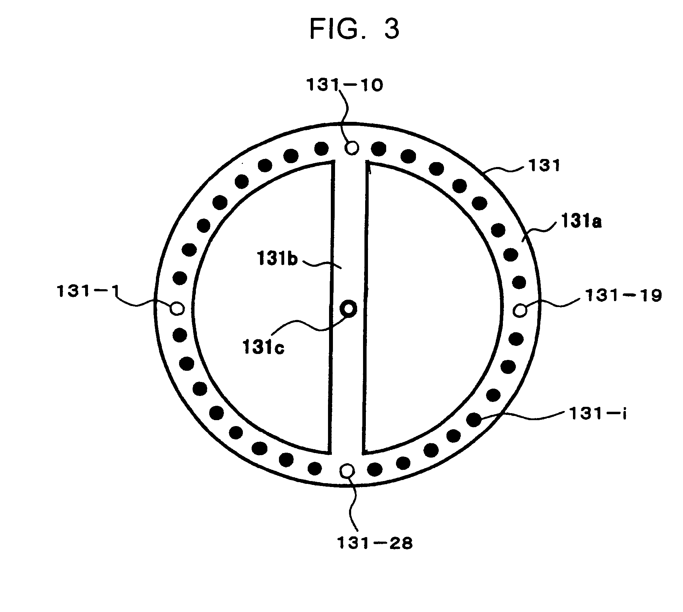

A configuration example of the projection image forming part 13 is shown in Fig. 2 and Fig. 3. The symbol 14 in Fig. 2 represents a photographing part. A TV camera 56, etc. that is discussed later is stored in the photographing part 14. Furthermore, Fig. 3 represents the configuration when a head part 131 of the projection image forming part 13 is viewed from the bottom (that is, from the side of the patient's eye E, in other words, from the side opposite to the lens tube part 10).

As indicated in Fig. 2 and Fig. 3, the head part 131 is a plate-like member. As indicated in Fig. 3, a toric periphery 131a and a fixation-light-source holder 131b, which is bridged in the direction of the diameter of the periphery 131 a, are provided in the head part 131.

Multiple LEDs 131-i (i=1 to N) are provided on the bottom surface of the periphery 131a. A group of LED 131-i are placed in a ring. In this embodiment, 36 LEDs 131-i are provided at even intervals (N=36). That is, the group of LEDs 131-i are placed at intervals of 10 degrees with regard to the center position of the group of LEDs 131-i. In other words, when considering line segments that connect each LED 131-i and the center position, two line segments that relate to LED 131-i and the adjacent 131-(i+1) are crossed at the center position at an angle of 10°.

Each LED 131-i emits visible light. A group of LEDs 131-i can be composed such that all LEDs emit the same color and can also be composed to emit different colors. The latter case can be composed such that LEDs in the horizontal direction and the vertical direction in the group of LEDs 131-i output different colors from the LEDs in other directions. That is, it can be composed such that LEDs placed at astigmatic axis angles (astigmatic axis directions) 0 degrees, 90 degrees, 180 degrees, and 270 degrees (LED 131-1, 131-10, 131-19, 131-28 accordingly) output luminous flux of different colors from other LEDs 131-i (i≠1, 10, 19, 28). For example, red LEDs can be used for LEDs 131-1, 131-10, 131-19, 131-28 along with green LEDs for other LEDs 131-i (i≠1, 10, 19, 28). As a result, the horizontal direction and the vertical direction of the astigmatic axes can be easily identified.

Furthermore, only a part of LED 131-1, 131-10, 131-19, and 131-28 can be set up to output luminous flux of different colors from other LEDs. For example, only LED 131-i corresponding to a 0 degree angle can be composed to output different colors than other LEDs (i≠1).

In addition, it is not necessary that all of the LEDs 131-1, 131-10, 131-19, and 131-28 are composed to output luminous flux of the same color (red in the example above). For example, red LEDs can be used for LEDs 131-1 and 131-19 while white LEDs can be used for LEDs 131-10 and 131-28 and green LEDs can be used for other LEDs 131-i (i≠1, 10, 19, 28). As a result, the horizontal direction and the vertical direction of the astigmatic axes can be easily identified.

Moreover, instead of making the horizontal direction and vertical direction identifiable by an output color of a light source such as above, other configurations can have a similar effect. For example, changing the brightness of the output light can make the directions identifiable.

A fixation LED 131 c for fixating the patient's eye E is provided on the bottom surface of the fixation-light-source holder 131b. The fixation LED 131c emits visible light. The fixation LED 131c is, for example, placed at the above center position of the group of LEDs 131-i, which are placed in a ring.

The number of the fixation LED 131 c is not limited to 1 but any number can be provided. Furthermore, the fixation LED 131c can be composed so as to be movable.

Moreover, the light source used for the head part does not have to be an LED and any device that can output light may be used. Furthermore, the multiple light sources placed in a ring do not have to be placed at even intervals. Further, because Fig. 3 is a diagram of the bottom surface, the placement order of the group of LEDs 131-i is set up in the opposite direction (reverse direction) from the direction set up for a general astigmatic axis. As a result, corneal reflection lights of luminous fluxes that re output from the group of LEDs 131-i (Purkinje images) are observed or photographed as the direction set up for a general astigmatic axis.

In addition, though 36 light sources are placed in a ring in this embodiment, the quantity is also arbitrary. It should be noted that when measuring the astigmatic axis angle of the patient's eye E based on the Purkinje image of luminous fluxes that are output by the group of LEDs 131-i, it is desirable that the quantity of light sources, which can ensure precision and accuracy of the measurements, are provided. When adopting a configuration that does not measure the stigmatic axis angles, there is no limit to the quantity of light sources in this sense.

Furthermore, depending on the precision required when the operator visually recognizes the orientation of the astigmatic axis angle and the principal meridian of toric IOL, the quantity of light sources can be properly set. For example, because light sources are placed at 10 degree intervals in this embodiment, the astigmatic axis angles can be presented in units of at least 10 degrees. In order to present the astigmatic axis angles, etc. with higher precision, the quantity of light sources, which corresponds to the precision (for example in units of 5 degrees, then 72 light sources), should be provided. The same can be said for lower precision cases.

Furthermore, though multiple light sources (the group of LEDs 131-i) are composed individually in this embodiment, this is not a restriction. For example, a display device (such as LCD (liquid-crystal display)) is provided on the bottom surface of the head part 131 and similar functionality can be obtained by displaying multiple bright points with this display device. It should be noted that a "bright point" in this embodiment refers to a light source that can be treated as a point light source.

An objective lens part 16 is provided at the bottom end of the lens tube part 10. An objective lens 15 (as stated below) is stored in the objective lens part 16. The supporting member 17 is provided in the vicinity of the objective lens part 16. The supporting member 17 is formed laterally from the objective lens part 16.

At the tip 17a of the supporting member 17, a vertically extending open hole is formed. An arm 133 is inserted into this open hole. The arm 133 is slidable inside the open hole. As a result, the arm 133 can be moved vertically (direction shown by the arrow A pointing at both sides in Fig. 2) with regard to the tip 17a. Here, the microscope 6 side is the top and the patient's eye E side is the bottom.

An anti-drop part 134 is provided at the top of the arm 133. The anti-drop part 134 is a plate-like member with a diameter bigger than the caliber of the open hole. As a result, the anti-drop part 134 prevents the arm 133 from falling from the tip 17a.

A head connecting part 132 is provided at the bottom end of the arm 133. The head connecting part 132 connects the head part 131 to the arm 133 such that the surface with LEDs 131-i face the bottom.

With such a configuration, the head part 131, the head connecting part 132, the arm 133, and the anti-drop part 134 (that is, the projection image forming part 13) can move freely and vertically with regard to the tip 17a. The projection image forming part 13 is moved by, for example, a user holding the arm 133, etc. Furthermore, by using a driving means such as a motor, the projection image forming part 13 can be composed such that it is moved electrically.

A connection hook 18 is provided on the bottom surface of the supporting member 17. The connection hook 18 is composed such that it can be engaged to an engaging part (not shown) of the projection image forming part 13. This engaging part is, for example, provided in the head connecting part 132. When the projection image forming part 13 is moved to the top, the engaging part engages the connection hook 18 to stop the vertical movement of the projection image forming part 13. This engagement relationship can be released by a specified operation (for example by pressing a specified button). The configurations of the connection hook 18 and the engaging part are optional.

With the above configuration, the group of LEDs 131-i is maintained such that they can move in the direction of the optical axis of the objective lens 15. Furthermore, the fixation 131 c is placed in the optical axis of the objective lens 15.

### [Configuration of the optical system]

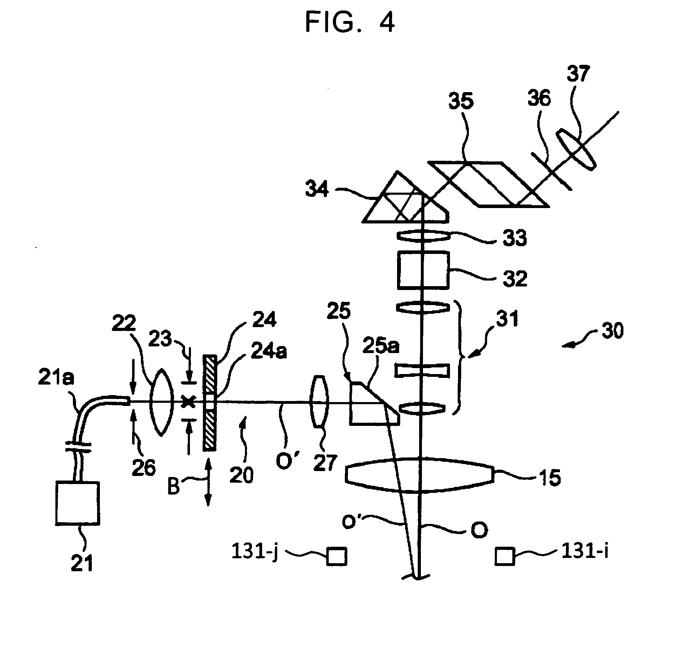

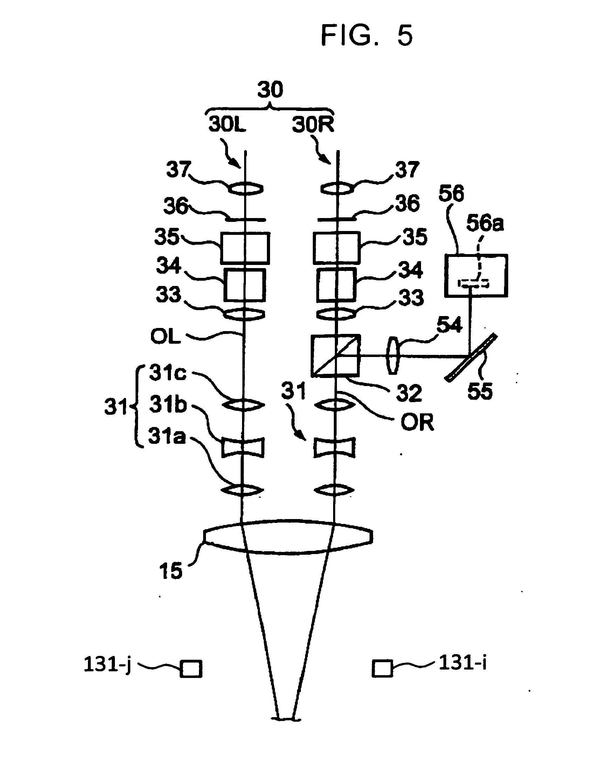

The optical system of the microscope for ophthalmologic surgery 1 will be explained next, with reference to Fig. 4 and Fig. 5. Here, Fig. 4 is a diagram, in which the optical system is viewed from the operator's left side. Moreover, Fig. 5 is a diagram, in which the optical system is viewed from the operator side. It should be noted that in addition to the configurations indicated in Fig. 4 and Fig. 5, an optical system (microscope for an assistant) for an assistant of the operator to observe the patient's eye E can be provided.

In this embodiment, directions such as top-and-bottom, left-and right, and front-and-back are directions viewed from the operator side unless otherwise specified. Moreover, with regards to the vertical direction, the direction from the objective lens 15 to the observation object (patient's eye E) is considered the lower direction while the opposite direction is considered the upper direction. Because patients generally go through an operation facing up, the top-and-bottom direction and the vertical direction become the same.

The group of LEDs 131-i is provided at the lower position of the objective lens 15 (that is, at the position between the objective lens 15 and the patient's eye E). Only LED 131-i and 131-j (i, j=1 to N, i≠j), which are positioned at both ends when viewed from the direction of this view point, are indicated in Fig. 4 and Fig. 5.

Moreover, a gap between the objective lens 15 and the patient's eye E means a gap between the position (height) of the objective lens 15 and the position (height) of the patient's eye E vertically (thus positions in the horizontal and front-and-back directions are not considered). Though the group of LEDs 131-i is placed in a ring, the diameter of this ring can be set arbitrarily as long as the size of images of the light (bright point images) from the entire group of LEDs 131-i is projectable to the cornea of the patient's eye E.

An observation optical system 30 will be explained. A symmetrical pair of observation optical systems 30 is provided as indicated in Fig. 5. The observation optical system 30L on the left will be referred to as a left observation optical system and the observation optical system 30R will be referred to as a right observation optical system. A symbol OL indicates an optical axis (observation optical axis) of the left observation optical system 30L and a symbol OR indicates an optical axis (observation optical axis) of the right observation optical system 30R. The observation optical systems 30L and 30R on the left and right are arranged such that an optical axis O of the objective lens 15 is located in the middle of the optical axes OL and OR.

As before, each of the observation optical systems 30L and 30R on the left and right has a zoom lens system 31, a beam splitter 32 (only the right observation optical system 30R), an imaging lens 33, an image-erecting prism 34, a pupil-distance-adjusting prism 35, a field diaphragm 36, and an eyepiece lens 37.

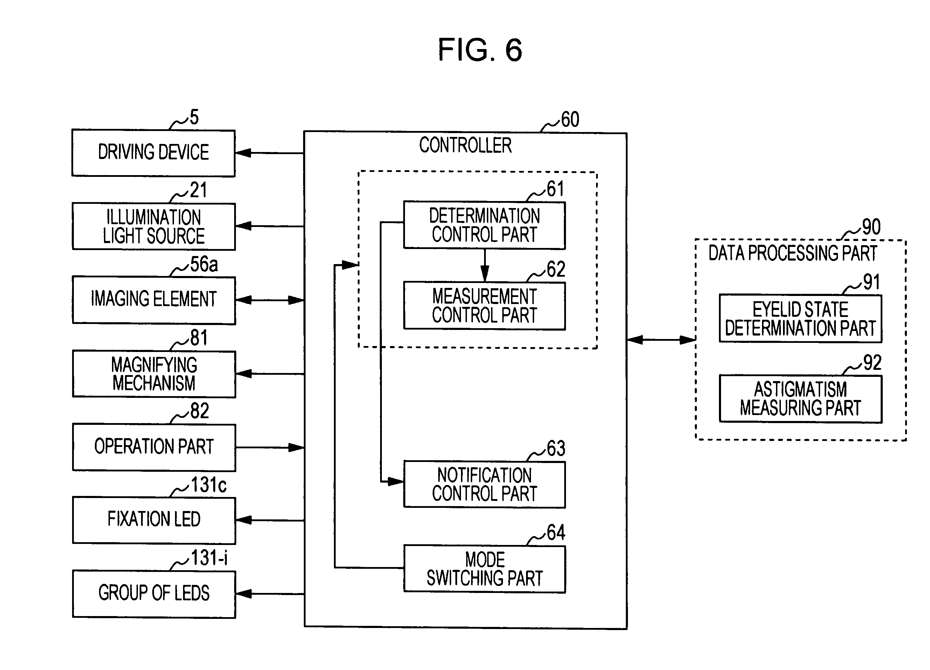

The zoom lens system 31 contains multiple zoom lenses 31a, 31b, and 31 c. Each zoom lens 31 a to 31 c is movable in the direction of the observation optical axis OL (or the observation optical axis OR) by the magnifying mechanism 81 that will be mentioned later (refer to Fig. 6). As a result, the magnification is changed when observing or photographing the patient's eye E. The zoom lens system 31 is an example of a "variable magnification optical system."

The beam splitter 32 of the right observation optical system 30R separates the part of the observation light that has been guided along the observation optical axis OR from the patient's eye E and leads to the TV camera imaging system. The TV camera imaging system comprises an imaging lens 54, a reflecting mirror 55, and a TV camera 56. The TV camera imaging system is stored in the photographing part 14.

The TV camera 56 is equipped with an imaging element 56a. The imaging element 56a is, for example, composed of a CCD (Charge Coupled Devices) image sensor, CMOS (Complementary Metal Oxide Semiconductor) image sensor, etc. As the imaging element 56a, an element that has a two-dimensional light-receiving surface (that is, an area sensor) is used.

When using the microscope for ophthalmologic surgery 1, the light-receiving surface of the imaging element 56a is placed, for example, at an optically conjugated position with the corneal surface of the patient's eye E or at an optically conjugated position that is deep from the corneal apex by half of a corneal curvature radius.

The image-erecting prism 34 converts a reversed image to an erect image. The pupil-distance-adjusting prism 35 is an optical element for adjusting the distance between left and right observation lights in accordance with the pupil distance of the operator (the distance between left and right eyes). The field diaphragm 36 restricts the operator's field of vision by defilading the surrounding area in a cross-section of the observation light.

An illumination optical system 20 is explained next. As indicated in Fig. 4, the illumination optical system 20 comprises an illumination light source 21, an optical fiber 21a, an emission diaphragm 26, a condensing lens 22, an illumination field diaphragm 23, a slit plate 24, a collimator lens 27, and an illumination prism 25.

The illumination field diaphragm 23 is provided at a position that is optically conjugate to the front focal position of the objective lens 15. Furthermore, a slit hole 24a of the slit plate 24 is formed at the optically conjugate position against the front focal position.

The illumination light source 21 is provided outside the lens tube part 10 of the microscope 6. One end of the optical fiber 21a is connected to the illumination light source 21. The other end of the optical fiber 21 a is placed at a position facing the condensing lens 22 inside the lens tube part 10. Illumination light that is output from the illumination light source 21 enters the condensing lens 22 after being guided by the optical fiber 21 a.

The emission diaphragm 26 is provided at a position facing the exit end (fiber edge on the condensing lens 22 side) of the optical fiber 21a. The emission diaphragm 26 works such that some area of the exit end of the optical fiber 21a is covered. When the covered area is changed by the emission diaphragm 26, the exit area of the illumination light is changed. As a result, for example, the projection angle of the illumination light, that is the angle formed by the incident direction of the illumination light toward the patient's eye E and the optical axis O of the objective lens 15, can be changed.

The slit plate 24 is formed by a discoidal member with a light blocking effect. A transparent part that is composed of multiple slit holes 24a with shapes corresponding to the shape of the reflective surface 25a of the illumination prism 25 is provided on the slit plate 24. The slit plate 24 is moved in a direction orthogonal to an illumination optical axis O' (in the direction shown by the arrow B pointing at both sides in Fig. 4) by a drive mechanism (not drawn). As a result, the slit plate 24 is inserted into and removed from the illumination optical axis O'.

The collimator lens 27 turns the illumination light that has passed through the slit hole 24a into parallel luminous flux. The illumination light that has been turned into the parallel luminous flux is reflected by the reflective surface 25a of the illumination prism 25 and projected onto the patient's eye E via the objective lens 15. (Some of) the illumination light projected onto the patient's eye E is reflected by the cornea. The reflected light (sometimes referred to as observation light) of the illumination light by the patient's eye E enters the observation optical system 30 via the objective lens 15. Due to such a configuration, a magnified image of the patient's eye E becomes observable.

### [Configuration of the control system]

The control system of the microscope for ophthalmologic surgery 1 will be explained with reference to Fig. 6. Moreover, the control system is partially shown in Fig. 6. The drive mechanism for the slit plate 24, etc. is omitted.

### (Controller)

The control system of the microscope for ophthalmologic surgery 1 is composed around the controller 60. The controller 60 is provided at an arbitrary part of the microscope for ophthalmologic surgery 1. Furthermore, aside from the configuration indicated in Fig. 1, a computer and/or a circuit board can be provided and used as the controller 60. The controller 60 is composed of a microprocessor and a storage device similar to a normal computer.

The controller 60 controls each part of the microscope for ophthalmologic surgery 1. In particular, the controller 60 controls the driving device 5, the illumination light source 21, the magnifying mechanism 81, the group of LEDs 131-i, the fixation LED 131c, etc. The driving device 5 moves the microscope 6 3-dimensionally after being controlled by the controller 60. The magnifying mechanism 81 moves each of the zoom lenses 31a, 31b, and 31c of the zoom lens system 31 after being controlled by the controller 60. For example, a pulse motor is provided in the driving device 5 and the magnifying mechanism 81. The controller 60 controls the motion by sending pulse signals to each pulse motor.

Moreover, signals are input into the controller 60 from the imaging element 56a and the operation part 82. Then, the controller 60 executes processes corresponding to the input signals based on computer programs and data (not shown).

The controller 60 is provided with a determination control part 61, a measurement control part 62, a notification control part 63, and a mode switching part 64.

### (Determination control part)

The determination control part 61 performs control related to determinations of the eyelid state of the patient's eye E. The eyelid state refers to the open or closed state of the eyelids, and more specifically refers to whether the eyelids of the patient's eye are in an open state (open-eyelid state) or a closed state (closed-eyelid state). A specific example of the process of determining the eyelid state is described later. The determination control part 61 is an example of a "determination part".

### (Measurement control part)

The measurement control part 62 performs control related to astigmatism measurement of the patient's eye E. Details are described later, but the measurement control part 62 controls the LEDs 131-i at a prescribed timing and executes astigmatism measurement. The prescribed timing refers to a prescribed time after it is determined that the eyelids have shifted from a closed state to an open state. For this reason, the determination control part 61 (or the measurement control part 62) is provided with a means for timing the prescribed time. For the timekeeping means, a timer installed on a microprocessor is used, for example.

Moreover, as the prescribed time (i.e., the threshold timekeeping time), the time required for the state of the fluid (tear fluid, etc.) on the corneal surface to settle is set in advance. This "settle" refers to, for example, the fluid regularly spreading to the corneal surface to a degree that does not adversely affect astigmatism measurement. More specifically, when a person blinks, tear fluid is supplied to the corneal surface, but at the initial stages of supply, the fluid is not spreading regularly to the corneal surface. As time passes from the blink, tear fluid is spreading gradually and regularly to the corneal surface. The time required for this is, for example, around 5 seconds. The threshold timekeeping time is set arbitrarily and may be, for example, determined statistically based on multiple items of data acquired clinically, or determined appropriately based on user experience, or determined individually based on past examination data of the patient's eye E.

### (Notification control part)

The notification control part 63 executes notification based on the determination results of the eyelid state. This notification is for informing the user that the time is unsuitable for performing astigmatism measurement of the patient's eye E, and/or that the eyelid state has continued to be the closed-eyelid state. Details of the notification process are described later. Together with the device being controlled, the notification control part 63 configures a "notification part". An example of this device is the LEDs 131-i. Moreover, it is also possible to perform notification by displaying prescribed information on a display device (not shown).

### (Mode switching part)

The mode switching part 64 performs switching of the astigmatism measurement mode. The astigmatism measurement modes include a first mode that performs astigmatism measurement based on the determination results of the eyelid state, and a second mode that performs astigmatism measurement in accordance with user instructions. Switching of the operation mode is realized by controlling the determination control part 61 and the measurement control part 62 as well as the astigmatism measuring part 92. Details of the mode switching re described later.

### (Operation part)

The operation part 82 is used by an operator, etc. to operate the microscope for ophthalmologic surgery 1. The operation part 82 includes all types of hardware keys (buttons, switches, etc.) that are provided in a case, etc. of the microscope 6 as well as the foot switches 8. Furthermore, when a touch panel display and GUI are provided, various types of software keys displayed on these are included in the operation part 82.

The operation part 82 includes an astigmatism measurement trigger switch. The trigger switch may be a hardware key as described above, the footswitch 8, or a software key. The second mode described above is an operation mode that performs astigmatism measurement in accordance with operation of the trigger switch.

### (Data processing part)

A data processing part 90 executes various types of data processing. The data processing includes a process of determining the eyelid state of the patient's eye E, and a process of obtaining astigmatism information of the patient's eye E. The astigmatism information includes at least the astigmatic axis direction, and may include the astigmatic degree. The data processing part 90 is provided with the eyelid state determination part 91 and the astigmatism measuring part 92.

### (Eyelid state determination part)

Under the control of the determination control part 61, the eyelid state determination part 91 determines the eyelid state of the patient's eye E, that is, whether or not the patient's eye E is in the open-eyelid state or the closed-eyelid state. The eyelid state determination part 91 is an example of a "determination part". A specific example of the process of determining the eyelid state is described below.

Determination of the eyelid state is executed by determining whether or not images of the multiple bright points (multiple bright point images) are depicted within prescribed regions in the frames of photographed images of the patient's eye E while the luminous fluxes from the LEDs 131-i are projected thereto. This photographed image is a moving image acquired at a prescribed frame rate by the imaging element 56a. Moreover, the luminous fluxes from the LEDs 131-i are visible lights. Each frame (image data) of the photographed image is input into the eyelid state determination part 91 via the controller 60 (determination control part 61). The eyelid state determination part 91 executes the following types of processing for each of the multiple frames that are input sequentially.

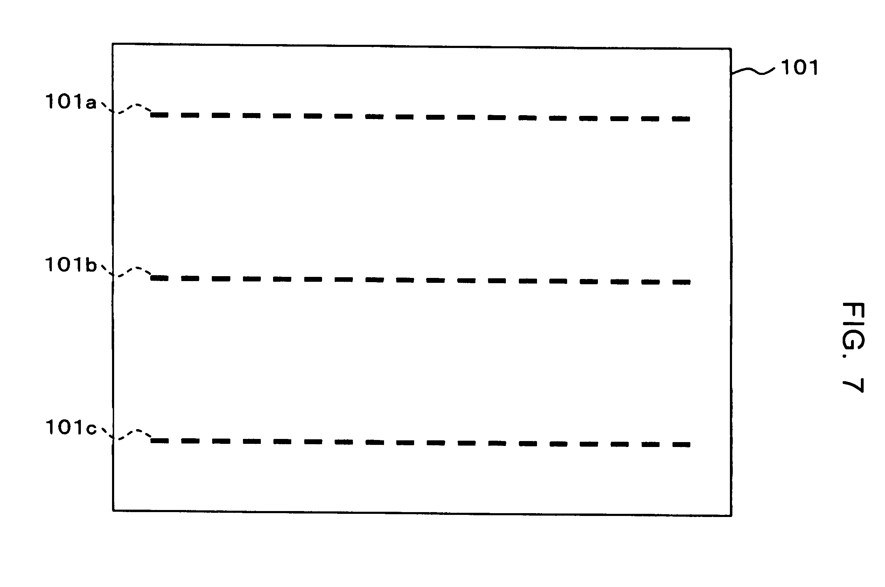

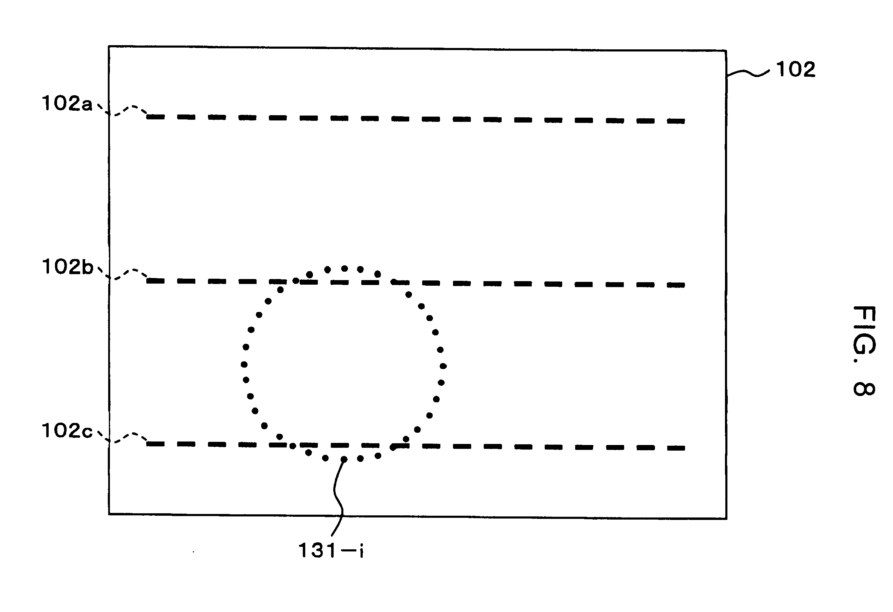

Examples of frames are shown in Fig. 7 and Fig. 8. In the frame 101 shown in Fig. 7, there is no bright point image, and in the frame 102 shown in Fig. 8, there are bright point images 131-i. The bright point images are images of corneal reflection of the LEDs 131-i and are indicated with the same symbol as the LEDs 131-i. In Fig. 7 and Fig. 8, depictions of images of the patient's body parts (the patient's eye E, the eyelids, etc.) are omitted.

As described above, the eyelid state determination part 91 analyzes prescribed regions within the input frames. As an example of the prescribed regions, in this embodiment, linear regions arranged in parallel at prescribed intervals are analyzed. The interval between the linear regions can be determined based on the positions at which the bright point images are depicted within the frame. More specifically, the interval between the linear regions can be set to a value equal to or less than the diameter of the ring-shaped arrangement of the multiple bright point images 131-i. This setting can be made by actually projecting the bright point images 131-i to a human eye or a schematic eye, or can be made based on a theory or a simulation. As an example of the latter, it is possible to determine the interval between the linear regions based on the imaging magnification of the zoom lens system 31. When doing this, it is also possible to reference the diameter of the ring-shaped arrangement of the LEDs 131-i or the distance between the LEDs 131-i and the patient's eye E (or the distance between a head part 131 and the microscope 6, which corresponds to that distance).

In this embodiment, for each frame, three linear regions extending in the horizontal direction are analyzed. For example, for the frame 101 shown in Fig. 7, each of the linear regions 101a, 101b, 101c are analyzed, and for the frame 102 shown in Fig. 8, each of the linear regions 102a, 102b, 102c are analyzed.

Based on the distribution of pixel values (e.g., luminance values) in the linear regions in the frame, the eyelid state determination part 91 determines whether or not bright point images are depicted in the frame. It should be noted that the luminous fluxes from the LEDs 131-i are reflected efficiently by the cornea but it not reflected very much by the eyelids. Consequently, if the bright point images 131-i are depicted in the frame, this indicates that the image shows the open-eyelid state, and conversely, if the bright point images 131-i are not depicted, this indicates that the image shows the closed-eyelid state. The following describe examples of processes of analyzing each of the frames 101 and 102.

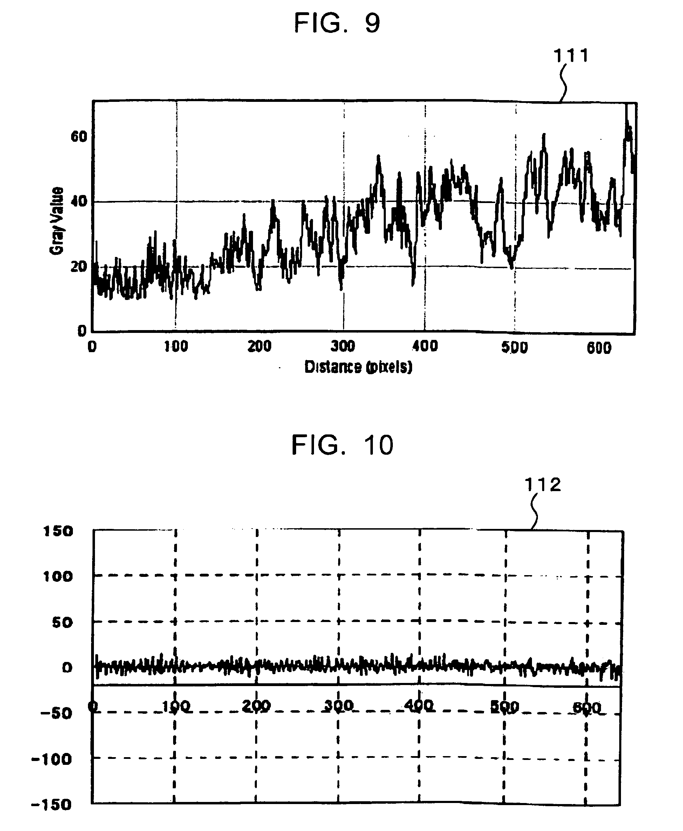

In the frame 101 shown in Fig. 7, the bright point images 131-i are not depicted. Fig. 9 shows an example of a luminance distribution 111 in the linear region 101a. The eyelid state determination part 91 obtains the luminance distribution 111 by plotting pixel information (coordinates and luminance values) of the linear region 101a in a coordinate system wherein the horizontal coordinates (pixel positions or pixel identification information) in the linear region 101 a are set as the horizontal axis and the luminance value of each pixel is set as the vertical axis. The same applies for the other linear regions 101b and 101c.

Furthermore, by differentiating the luminance distribution 111 with respect to the horizontal-axis coordinates, the eyelid state determination part 91 obtains the luminance gradient distribution 112 (derivatives) shown in Fig. 10. This differentiation process corresponds to an edge enhancement process. Consequently, it is also possible to apply an edge enhancement process other than the differentiation process.

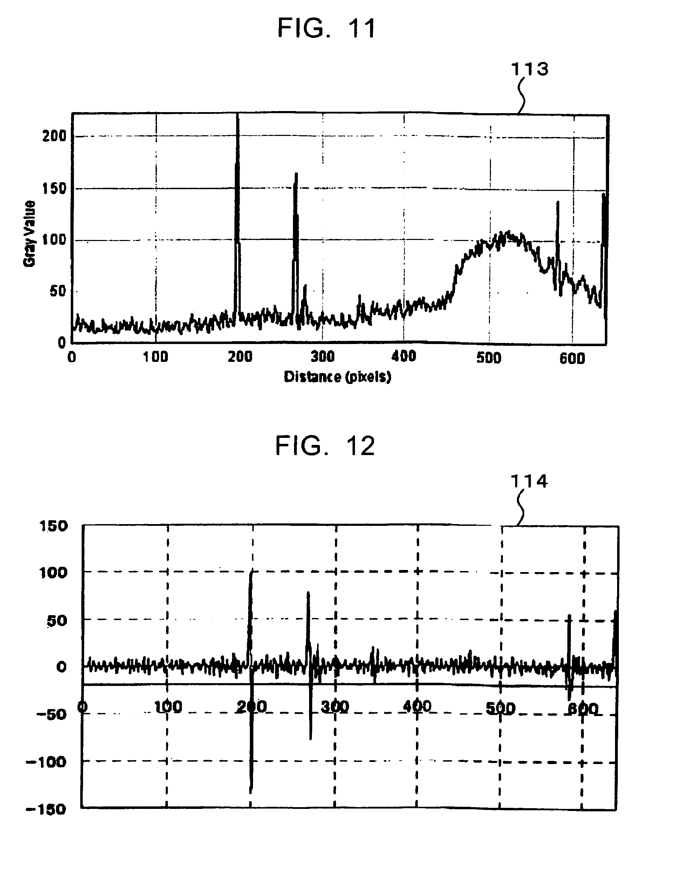

Similarly, for the frame 102 shown in Fig. 8, the eyelid state determination part 91 obtains the luminance distribution and luminance gradient distribution in each of the linear regions 102a-102c. The luminance distribution 113 shown in Fig. 11 is based on the linear regions intersecting with the bright point images 131-i (in this case, the linear region 102b or 102c), and the luminance gradient distribution 114 shown in Fig. 12 is obtained by differentiating the luminance distribution 113.

As is clear by comparing the luminance gradient distribution 112 of Fig. 10 and the luminance gradient distribution 114 of Fig. 12, in the latter, there is a peak that is not present in the former. This peak indicates that the luminance values of the adjacent pixels change rapidly and that therefore, the bright point images 131-i are depicted there.

As an example of a process for detecting such a peak, the eyelid state determination part 91 executes a thresholding process. The thresholding process determines whether or not there is a gradient (derivative) of luminance values exceeding a prescribed threshold value (e.g., an absolute gradient of 50).

The peak detection process is not limited to this, and the specific processing details may be of any kind as long as it can detect the peak of the luminance gradient distribution graph. Moreover, it is also possible to determine whether or not the bright point images 131-i are depicted based on the luminance distribution itself, without obtaining the luminance gradient distribution. For example, as is clear from comparing Fig. 9 and Fig. 11, because the two graphs have different shapes, it is possible to apply any process that can detect this difference in shape. Differences in shape include the presence or absence of a peak as well as frequency, etc.

The eyelid state determination part 91 executes the above processes on frames that are input sequentially and determines whether or not the bright point images 131-i are depicted in each frame, and sequentially sends the determination results to the determination control part 61. This process is executed in practically real time.

Based on the determination results input sequentially from the eyelid state determination part 91, the determination control part 61 recognizes the eyelid state of the patient's eye E in practically real time. Furthermore, upon recognizing that the eyelids have shifted from a closed state to an open state, the determination control part 61 starts the timekeeping performed by the timer described above. Then, based on the time being kept by the timer and on the determination results of the eyelid state input sequentially from the eyelid state determination part 91, the determination control part 61 determines whether or not the open-eyelid state has continued for a prescribed time.

If the open-eyelid state has not continued for a prescribed time, the eyelid state determination part 91 stands by until the open-eyelid state occurs again, and restarts the timekeeping performed by the timer from the beginning when the open-eyelid state occurs again.

If a determination is made that the open-eyelid state has continued for a prescribed time, the determination control part 61 sends an instruction to start measurement to the measurement control part 62. The measurement control part 62 receives this instruction and controls the astigmatism measuring part 92, etc. to execute astigmatism measurement. Furthermore, in this embodiment, the LEDs 131-i are on continuously, and it is therefore not necessary to perform lighting control at this time. However, if the lighting mode of the LEDs 131-i is to be changed between the process of determining the eyelid state and the astigmatism measurement, controls are performed to do so.

### (Astigmatism measuring part)

Based on photographed images of the patient's eye E in a state in which the luminous fluxes from the LEDs 131-i are projected onto the cornea, the astigmatism measuring part 92 calculates the astigmatic axis direction of the patient's eye E. As with a conventional keratometer, this process is executed based on the arrangement of the multiple bright point images 131-i (e.g., the axis direction of the elliptic arrangement). Moreover, for the process for calculates the astigmatic degree, as with a conventional keratometer, this process is also executed based on the arrangement of the multiple bright point images 131-i (e.g., the ellipticity). The astigmatism measuring part 92 is an example of the "calculation part".

### [Actions]

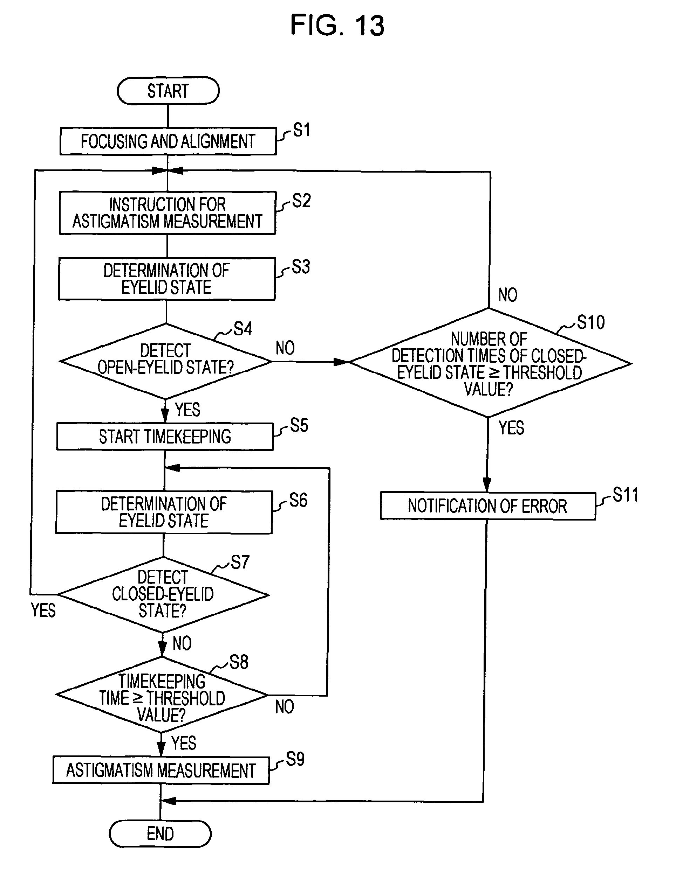

Actions of the microscope for ophthalmologic surgery 1 are described. Fig. 13 shows an example of the action thereof.

### (S1)

First, the user performs focusing and alignment of the microscope optical system with respect to the patient's eye E. These procedures are executed in a conventional manner, for example.

### (S2)

Once the focusing and alignment are completed, the user issues an instruction for astigmatism measurement. This instruction is issued by, for example, operating the trigger switch described earlier. The controller 60 receives a signal from the trigger switch and starts up the determination control part 61 and also turns on the LEDs 131-i. At prescribed time intervals, a TV camera 56 photographs the patient's eye E while luminous fluxes from the LEDs 131-i are projected thereto. As a result, a moving image of the patient's eye E is obtained. The frames of the moving image are input sequentially into the controller 60.

### (S3)

The eyelid state determination part 91 analyzes the frames that are input sequentially and determines whether the eyelids are in an open state or a closed state.

### (S4 Yes, S5, S6)

When it is determined that the patient's eye E are in the open-eyelid state (e.g., when it is determined that the eyelids have shifted from the closed state to the open state), the determination control part 61 starts timekeeping. This timekeeping is performed, for example, by counting up until a prescribed time or counting down from a prescribed time. The threshold value for this timekeeping time is, as described earlier, the time required for tear fluid to spread regularly to the corneal surface.

In parallel with this timekeeping, the eyelid state determination part 91 determines whether the eyelids are in the open state or the closed state.

### (S7 No, S8, S9)

If, after shifting from the closed-eyelid state to the open-eyelid state, the open-eyelid state has continued for the prescribed time (i.e, if the timekeeping time has reached the threshold value with no detection of the closed-eyelid state after the detection of the open-eyelid state), the determination control part 61 sends an instruction to perform measurement to the measurement control part 62. The measurement control part 62 controls the astigmatism measuring part 92 and calculates the astigmatism information of the patient's eye E. In this case, the processes end here.

### (S7 Yes)

If it is detected that the closed-eyelid state has occurred before the timekeeping time reaches the threshold value, the process returns to step 2 and waits for the shift to the open-eyelid state. Error processing may be performed if a "Yes" result repeatedly occurs a prescribed number of times in step 7. The error processing may be similar to that described later.

### (S4 No, S10, S11)

If the open-eyelid state does not occur, there is a risk that a problem for performing astigmatism measurement, such as the patient's eye E remaining closed or a device malfunction, has occurred. The determination control part 61 counts the time during which the closed-eyelid state continues, for example by counting the number of times the closed-eyelid state is detected consecutively. Then, the determination control part 61 repeats steps 2 through 4 until this number reaches a prescribed threshold value.

When the number reaches the threshold value, the determination control part 61 sends an instruction to the notification control part 63. Upon receiving this instruction, the notification control part 63 executes a prescribed error notification operation. This error notification operation is for notifying the user that the astigmatism measurement cannot be executed, and specific examples include methods such as making (at least one of) the LEDs 131-i blink, making the fixation LED 131 c blink, or displaying prescribed information on a display device (not shown). Upon recognizing the occurrence of the error, the user confirms the state of the patient and the device or confirms the state of the alignment and focus. With the above, the operational description based on the flowchart shown in Fig. 13 is complete.

### (Changes in timekeeping time)

The user can change the threshold value for the above timekeeping time by taking into consideration the state of the patient, etc. This process is executed by performing prescribed operations using the operation part 82. The operation part 82 is an example of a "changing part".

### (Switching operation modes)

The user can freely divide usage of an operation mode (first mode) that uses the eyelid state as a trigger for astigmatism measurement and an operation mode (second mode) in which the user directly inputs a trigger for astigmatism measurement. Selection of the operation mode is executed by performing prescribed operations using the operation part 82, for example. The mode switching part 64 sends an instruction to the determination control part 61 and the measurement control part 62 to execute the selected operation mode. The first mode is the operation mode described in this embodiment, and the second mode is an operation mode similar to conventional examples.

### [Effects]

Effects of the microscope for ophthalmologic surgery 1 are described.

As with a typical device, the microscope for ophthalmologic surgery 1 has an optical system that photographs the patient's eye E as well as the microscope 6 that stores at least part of the optical system. Furthermore, the microscope for ophthalmologic surgery 1 comprises the projection image forming part 13, the eyelid state determination part 91, and the astigmatism measuring part 92. The projection image forming part 13 is attached to the microscope 6 and has multiple bright points arranged in a ring. The eyelid state determination part 91 determines whether or not bright point images are depicted within prescribed regions in the frame of an image (first image) obtained by using the optical system to photograph the patient's eye E while multiple bright points are projected thereto, and thereby determines whether the eyelids of the patient's eye E are open or closed. The astigmatism measuring part 92 calculates the astigmatism information of the patient's eye E based on an image (second image) obtained by using the optical system to photograph the patient's eye E while multiple bright points are projected thereto.

According to this microscope for ophthalmologic surgery 1, because it is possible to detect whether or not the eyelids of the patient's eye E are open or closed, it is possible to properly perform astigmatism measurement while the eyelids are definitely in an open state. Moreover, because the user can perform astigmatism measurement at a suitable time once the eyelids shift from the closed state to the open state, it is possible to eliminate the effects of the state of the cornea of the patient's eye, particularly the state of tear fluid, etc. on the corneal surface, on the corneal reflection of the bright points, and it is possible to perform astigmatism measurements correctly.

Moreover, when the determination control part 61 and the eyelid state determination part 91 determine that the eyelids have shifted from the closed state to the open state and that the open-eyelid state has continued for a prescribed time, the microscope for ophthalmologic surgery 1 operates to obtain the astigmatism information of the patient's eye E based on images obtained by using the optical system to photograph the patient's eye E while multiple bright points are projected thereto.

As a result, it is possible to perform astigmatism measurement automatically when the state of the cornea of the patient's eye, and particularly the state of tear fluid, etc. on the corneal surface, do not substantially affect the corneal reflection of the bright points. Consequently, there is the effect that it is possible to perform astigmatism measurement automatically at a proper timing.

Moreover, with the microscope for ophthalmologic surgery 1, it is possible to selectively execute the operation mode described in this embodiment (first mode) or the conventional operation mode (second mode). Consequently, there is the advantage that it is possible to appropriately select an operation mode according to the user's needs.

Moreover, the microscope for ophthalmologic surgery 1 is configured to determine the eyelid state at prescribed time intervals and also to provide notification when determination results indicating the closed-eyelid state are consecutively obtained a prescribed number of times. In this way, because it is possible to notify the user that a situation preventing proper astigmatism measurement has occurred, the user is able to swiftly perform suitable measures.

### <Modification example>

The details described above are nothing more than one embodiment of the present invention. Persons implementing the present invention may implement any modifications (including omissions and additions, etc.) within the scope of the spirit of the present invention.

For example, the process for determining the eyelid state is not limited to that described above. As a specific example, instead of the LEDs 131-i, it is also possible to use the fixation LED 131c as a bright point, or to use other light sources as bright points.

Moreover, it is possible to analyze the frames of moving images of the patient's eye E to detect the depicted state of the patient's eye E and/or characteristic parts in its vicinity, and to determine the eyelid state based on those detected results. For example, by determining whether or not the pupils of the patient's eye E are depicted, it is possible to determine that the eyelids are open if the pupil is depicted or that the eyelids are closed if the pupil is not depicted. Here, it is also possible to reflect the size (region) of the pupil that is depicted. In addition, it is also possible to take into consideration the depicted state of parts such as the borders of the eyelids, etc.

### [Explanation of Symbols]

- 1: Microscope for ophthalmologic surgery
- 5: Driving device
- 13: Projection image forming part
- 20: Illumination optical system
- 21: Illumination light source
- 30: Observation optical system
- 56a: Imaging element
- 60: Controller
- 61: Determination control part
- 62: Measurement control part
- 63: Notification control part
- 64: Mode switching part
- 81: Magnifying mechanism
- 82: Operation part
- 90: Data processing part
- 91: Eyelid state determination part
- 92: Astigmatism measuring part
- 101, 102: Frame
- 101a, 101b, 101c, 102a, 102b, 102c: Linear region
- 111, 113: Luminance distribution
- 112, 114: Luminance gradient distribution
- 131-i: LED
- 131 c: fixation LED

## Claims

1. A microscope for ophthalmologic surgery comprising:
an optical system that photographs a patient's eye;
a main body part that stores at least part of the optical system;
an attachment that is attached to the main body part and has multiple bright points arranged in a ring;
a determination part that determines whether the eyelids of the patient's eye are open or closed by determining whether or not bright point images are depicted within a prescribed region in the frame of a first image obtained by using the optical system to photograph the patient's eye while the multiple bright points are projected thereto; and
a calculation part that calculates astigmatism information of the patient's eye based on a second image obtained by using the optical system to photograph the patient's eye while the multiple bright points are projected thereto.

2. A microscope for ophthalmologic surgery comprising:
an optical system that photographs a patient's eye;
a main body part that stores at least part of the optical system;
an attachment that is attached to the main body part and has multiple bright points arranged in a ring;
a determination part that determines whether the eyelids of the patient's eye are open or closed by determining whether or not bright point images are depicted within a prescribed region in the frame of a first image obtained by using the optical system to photograph the patient's eye while the multiple bright points are projected thereto; and
a calculation part that, when the determination part determines that the eyelids have shifted from a closed state to an open state and that the open-eyelid state has continued for a prescribed time, calculates astigmatism information of the patient's eye based on a second image obtained by using the optical system to photograph the patient's eye while the multiple bright points are projected thereto.

3. The microscope for ophthalmologic surgery according to Claim 1 or 2, wherein the prescribed regions include multiple linear regions arranged in parallel at a prescribed interval.

4. The microscope for ophthalmologic surgery according to Claim 3, wherein the prescribed interval is determined based on the positions at which the bright point images are depicted in the frame.

5. The microscope for ophthalmologic surgery according to Claim 3, wherein
the optical system includes a variable magnification optical system that changes the imaging magnification, and
the prescribed interval is determined based on the imaging magnification.

6. The microscope for ophthalmologic surgery according to any of Claim 1 through 5, wherein the determination part determines whether or not the bright point images are depicted based on the distribution of luminance values in the prescribed regions.

7. The microscope for ophthalmologic surgery according to Claim 6, wherein the determination part differentiates the distribution of the luminance values and determines whether or not the bright 'point images are depicted based on the distribution of the derivatives.

8. The microscope for ophthalmologic surgery according to any of Claims 1 through 7, wherein each of the multiple bright points outputs light including a wavelength of the visible region.

9. A microscope for ophthalmologic surgery comprising:
an optical system that photographs a patient's eye;
a main body part that stores at least part of the optical system;
an attachment that is attached to the main body part and has multiple bright points arranged in a ring;
a determination part that determines whether the eyelids of the patient's eye are open or closed based on images of the patient's eye photographed by the optical system; and
a calculation part that, when the determination part determines that the eyelids have shifted from a closed state to an open state and that the open-eyelid state has continued for a prescribed time, calculates astigmatism information of the patient's eye based on an image obtained by using the optical system to photograph the patient's eye while the multiple bright points are projected thereto.

10. The microscope for ophthalmologic surgery according to Claim 2 or 9, further comprising a changing part for changing the prescribed time.

11. The microscope for ophthalmologic surgery according to Claim 2 or 9, further comprising:
an operation part; and
a mode switching part that switches the operation mode of the calculation part between a first mode that calculates the astigmatism information based on determination results from the determination part, and a second mode that calculates the astigmatism information in accordance with operations of the operation part.

12. The microscope for ophthalmologic surgery according to any of Claims 1 through 11, wherein
the determination part performs the determination at a prescribed time interval, and
the microscope for ophthalmologic surgery further comprises a notification part that performs notification when determination results indicating the closed-eyelid state are continuously obtained a prescribed number of times by the determination part.
